(19) Europäisches Patentamt
European Patent Office
Office européen des brevets

(11) **EP 3 658 131 B1**

(12) **EUROPEAN PATENT SPECIFICATION**

(45) Date of publication and mention
of the grant of the patent:
**26.05.2021 Bulletin 2021/21**

(21) Application number: **18746848.3**

(22) Date of filing: **10.07.2018**

(51) Int Cl.:
*A61K 31/19* (2006.01)    *A61K 31/4725* (2006.01)
*A61K 31/716* (2006.01)    *A61K 47/12* (2006.01)
*A61P 15/02* (2006.01)    *A61K 9/02* (2006.01)
*A61K 9/06* (2006.01)    *A61K 9/08* (2006.01)
*A61K 9/00* (2006.01)    *A61K 47/36* (2006.01)

(86) International application number:
**PCT/EP2018/068685**

(87) International publication number:
**WO 2019/020367 (31.01.2019 Gazette 2019/05)**

(54) **COMPOSITION FOR THE TREATMENT OF BACTERIAL VAGINOSIS**

ZUSAMMENSETZUNG ZUR BEHANDLUNG VON BAKTERIELLER VAGINOSE

COMPOSITION POUR LE TRAITEMENT DE LA VAGINOSE BACTÉRIENNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.07.2017 IT 201700086482**

(43) Date of publication of application:
**03.06.2020 Bulletin 2020/23**

(73) Proprietor: **Farma-Derma S.R.L.
40010 Sala Bolognese, BO (IT)**

(72) Inventor: **RUSSO, Vincenzo
40137 Bologna (IT)**

(74) Representative: **Meissner Bolte Partnerschaft
mbB
Patentanwälte Rechtsanwälte
Postfach 86 06 24
81633 München (DE)**

(56) References cited:
**EP-A1- 1 481 666**

• **MENDLING WERNER ET AL: "Use of locally
delivered dequalinium chloride in the treatment
of vaginal infections: a review", ARCHIVES OF
GYNECOLOGY AND OBSTETRICS, SPRINGER
VERLAG, BERLIN, DE, vol. 293, no. 3, 27 October
2015 (2015-10-27), pages 469-484, XP035879005,
ISSN: 0932-0067, DOI:
10.1007/S00404-015-3914-8 [retrieved on
2015-10-27]**

**Description**

The present invention relates to a composition comprising a dequalinium salt, maltodextrin and lactic acid for application in the treatment of the bacterial vaginosis. The inventor proposes an improved therapeutic strategy based on the action synergy of the three components of the composition, whose antibacterial effect is greater than the sum of the effects exerted by the individual substances. Furthermore, the aforementioned composition is able to simultaneously promote the growth of the lactobacilli, naturally present in the vagina. The composition according to the invention is stable both in a liquid solution and in a solid or semi-solid solution. The preferred form of administration is via the vaginal route, in the form of a liquid solution, in solid form such as ovules or semi-solid such as cream.

PRIOR ART

The vagina is a female genital organ which, although internal, does not constitute an aseptic environment because of its connection with the outside. Furthermore, since its opening is close to the anus, it is a densely populated area where numerous populations of micro-organisms live peacefully together which, as a whole, constitute what is defined as "vaginal flora". Among these, a primary role is played by the lactobacilli (*Lactobacillus acidophilus*), which exert their protective function through the following mechanisms:

- they transform glycogen into lactic acid, the substance that has the key role of ensuring an acidic pH in the vaginal environment comprised between 3.5 and 4.5, which is unfavourable to the colonisation of pathogenic micro-organisms that could disturb the delicate balance of the ecosystem. The lowering of the pH caused by the lactic acid causes the rapid growth of the lactobacilli that contribute in turn to the production of lactic acid and, therefore, to further protecting the vaginal environment;
- they produce hydrogen peroxide ($H_2O_2$) which is toxic for most of the other vaginal bacterial species free from the catalysis detoxifying enzyme;
- they produce bactericidal substances (bacteriocins);
- by specifically and non-specifically binding to the epithelial cells of the vaginal mucosa they prevent the pathogenic micro-organisms finding free binding sites and, therefore, adhering to the vaginal mucosa.

The vaginal infections are diseases of the vagina caused by one or more infectious agents. The inflammation often also extends to the vulva and to other areas of the lower genital tract and, in this case, we talk about vulvovaginitis. The vaginal infections occur in 90% of cases in the fertile age and represent the main cause of demand for gynaecology appointments. Among the vaginal infections, the bacterial vaginosis (BV) represents the most common vaginal infection that bothers pregnant women, women of a fertile age and pre-menopausal women. The BV is a complex disorder characterised by a polymicrobism i.e. excessive growth of anaerobic bacteria (*Gardnerella vaginalis, Atopobium vaginae, Prevotella* spp., *Mobiluncus* spp., *Mycoplasma hominis,*) or of bacteria of intestinal origin (*Escherichia coli, Proteus* spp, *Klebsiella* spp, *Serratia* spp, *Staphylococcus aureus*) caused by an altered vaginal ecosystem with a reduction of the *Lactobacillus acidophilus,* in particular the one that produces the hydrogen peroxide. The BV is associated with an increase in vaginal pH (above 4.7), due to the reduction in lactic acid produced by the lactobacilli themselves, which creates the ideal condition for the development of pathogenic bacteria.

The BV is defined as a syndrome rather than an individual pathology since it is characterised by a string of different causes and symptoms. The conditions that can contribute pathologically to the destruction of the physiological vaginal ecosystem promoting the proliferation of pathogenic agents and vaginal symptoms are:

- inappropriate intimate hygiene;
- use of antibiotics that neutralise not only pathogenic organisms, but also "good" ones such as the *Lactobacillus Acidophilus;*
- chronic stress;
- hormonal contraceptives with low doses of ethinyl estradiol that cause a state of hypoestrogenism with the consequent interruption of the production of glycogen and, therefore, lactic acid.

In some cases, the BV is asymptomatic whereas in other cases women complain of discomfort and malodorous and abundant vaginal discharge characterised by the typical fish odour, caused by the volatilisation of amines (putrescine, cadaverine and trimethylamine) produced by the metabolism of anaerobic bacteria that characterise this disorder.

To date, the therapy options for the BV are as follows:

- use of antibiotics and/or local antiseptics that lead to the eradication of the microorganism involved;
- use of vaginal ecosystem rebalancing agents i.e. products that restore the vaginal ecosystem. They can be split into:

- substances that stimulate the proliferation of the lactobacilli (e.g. probiotics);
- acidifying substances that reduce the vaginal pH promoting the natural growth of the lactobacilli and hinder the proliferation of pathogens.

The use of wide spectrum antibiotics such as metronidazole or clindamycin represents the elected therapy for the treatment of the BV. Although antibiotics are very effective in reducing symptoms, they can cause some problems:

- frequent relapses;
- lack of treatment response;
- antibiotic resistance;
- post-treatment candidiasis.

Because of the problems mentioned above, the local antiseptics represent a valid alternative to the use of antibiotics particularly in the case of repeated chronic treatments or in the case of bacterial resistance. The ones most commonly used are chlorhexidine, povidone iodide, hydrogen peroxide, actenidina and polyhexamethylene biguanide (Haya J., Garcia A., López-Manzanara C., Balawi M., Haya L., "Importance of Lactic Acid in Maintaining Vaginal Health: A Review of Vaginitis and Vaginosis Etiopathogenic Bases and a Proposal for a New Treatment", Open Journal of Obstetrics and Gynecology, 2014, 4, 787-799; Carol A. Spiegel "Bacterial Vaginosis" Clinical Microbiology Reviews, Oct. 1991, p. 485-502).

IT2003TO00404, corresponding to EP1481666, discloses a composition suitable for rebalancing the vaginal ecosystem by reconstitution of an environment which favours the development of the lactobacilli, comprising lactic acid for regulating the pH and a nutrient support for the lactobacilli comprising maltodextrin. US3147182 discloses antibacterial compositions for controlling microbiological floras comprising in particular a salt of decamethylene 1,10-bis-4-aminoquinaldinium as an antimicrobial agent in an acidic environment preferably for acetates and chloride ions. The use of the composition includes among other things the bacteriological control of the oral cavity, gum paints and other special antiseptic uses such as vaginal irrigations and cold sterilisation solutions. GB1126953 discloses a bactericidal composition comprising at least one water-soluble quaternary ammonium surfactant cationic compound, an organic acid able to form a water-soluble derivative or derivatives with the quaternary ammonium compound or compounds, and a water-soluble non-ionic surfactant, in a carrier comprising an aqueous solution containing at least 30% by weight of ethanol or isopropanol. The cationic quaternary ammonium compound may be amongst other things a dequalinium acetate or a dequalinium chloride. The organic acid may be citric, malic, lactic, glycolic, tartaric, benzoic or succinic acid.

GB2450225, corresponding to application EP2152264, discloses an antimicrobial formulation containing a derivative of mercaptopyridine and a bis-quinolinium salt. The bis-quinolinium salt may be a dequalinium salt. The formulation can be used for treating a particular condition caused by a bacterial proliferation such as the acne or the body odour and is appropriately applied locally.

W. Mendling et al., "Use of locally delivered dequalinium chloride in the treatment of vaginal infections : a review", Archives of Gynecology and Obstetrics, Springer Verlag, Berlin, Germany, vol. 293, no. 3, publication date October 27, 2015, pages 469 to 484, discloses a variety of different medicinal products comprising dequalinium chloride currently available on the market, wherein none of the medicinal products contains a combination of dequalinium chloride in combination with lactic acid and maltodextrin.

The object of the present invention is to develop a new composition of substances that is suitable for use in treating bacterial vaginosis, as well as exerting an antibacterial effect, and to simultaneously promote the growth of the lactobacilli, naturally present in the vagina.

SUMMARY OF THE INVENTION

The present invention relates to a new composition containing a dequalinium salt, maltodextrin and lactic acid according to claim 1 for use in the treatment of bacterial vaginosis (BV).
The composition may be in the form of a liquid solution, solid form such as ovules or semi-solid such as cream.
The anion of the dequalinium salt may be selected from chloride, bromide, iodide, acetate and undecanoate; preferably chloride.
According to a preferred embodiment, the composition is a solid composition in the form of ovules of having a weight of 1.5 - 2.5 g, preferably ovules having a weight of 2.0 g, wherein preferably, the concentration by weight of dequalinium ion is between 0.85‰ - 13‰, more preferably 2.20‰ - 13‰, most preferably 3.45‰ - 9‰. According to this embodiment, the concentration by weight of maltodextrin is between 1.0‰ - 4.5‰, preferably 2.0‰ - 3.0‰; while the concentration by weight of lactic acid is between 1.0‰ - 20‰, preferably 4.0‰ - 6.0‰. Optionally, the ovules comprise one or more stable pharmaceutically acceptable excipients selected from semi-synthetic glycerides, silica dimethyl silylate, vitamin E acetate and butylhydroxytoluene; preferably the ovules have a weight of 2 g.

According to a preferred embodiment, the composition is an aqueous composition comprising one or more stable pharmaceutically acceptable excipients in disposable 100 ml bottles.

According to this embodiment the concentration by weight of dequalinium ion is preferably comprised between 0.017‰ - 0.26‰, the concentration by weight of maltodextrin is between 0.02‰ - 0.09‰, the concentration by weight of lactic acid is between 0.02‰ - 0.4‰; more preferably the concentration by weight of dequalinium ion is between 0.044‰ - 0.26‰, the concentration by weight of maltodextrin is between 0.04‰ - 0.06‰, and the lactic acid concentration by weight is between 0.08‰ - 0.12‰; most preferably the concentration by weight of dequalinium ion is between 0.069‰ - 0.18‰, maltodextrin between 0.04‰ - 0.06‰, and lactic acid is between 0.08‰ - 0.12‰. Preferably, the stable pharmaceutically acceptable excipients are selected from glycerin, PEG 40 hydrogenated castor oil, chlorhexidine digluconate, sodium benzoate, lavender essential oil.

According to a preferred embodiment, the composition is a cream packaged in 30 g tubes with 6 disposable applicators. In the cream composition, the concentration by weight of dequalinium ion is preferably comprised between 0.34‰ - 5.2‰, the concentration by weight of maltodextrin is between 0.4‰ - 1.8‰, the concentration by weight of lactic acid is comprised between 0.4‰ - 8‰; more preferably the concentration by weight of dequalinium ion is between 0.88‰ and 5.2‰, the concentration by weight of maltodextrin is between 0.8‰ - 1.2‰, the lactic acid concentration by weight is between 1.6‰ - 2.4‰; most preferably the concentration by weight of dequalinium ion is between 1.38‰ - 3.6‰, maltodextrin between 0.8‰ - 1.2‰, and lactic acid is between 1.6‰ - 2.4‰. The stable pharmaceutically acceptable excipients of the cream are preferably selected from one or more excipients selected from glyceryl stearate, PEG 100 stearate, cetylstearyl alcohol, fatty acid esters, cyclopentasiloxane, vegetable stearic acid, chlorhexidine digluconate, silicone oil, triethanolamine, EDTA bisodic salt, carbomer and other lipophilic excipients.

The term "concentration by weight" as used herein for any one of the above compositions and used in relation to any one of the components dequalanium ion, maltodextrin and lactic acid means that the respective component is contained in the composition in a ratio of mass of the component to the total mass of the composition, given in permille (‰).

Each of the aforesaid substances performs the therapeutic effect through one of the mechanisms of action mentioned above recognised as being effective for the eradication of the BV.

In particular,

- dequalinium salt: performs a local disinfectant action;
- maltodextrins: represent the nutritious substrate adapted to promote the growth of the endogenous lactobacilli;
- lactic acid: restores the acid pH in the vagina creating a favourable environment for the growth of the lactobacilli with the consequent inhibition of the proliferation of the pathogenic micro-organisms.

The inventor proposes an improved therapeutic strategy based on the action synergy of the aforementioned ingredients of the composition, whose antibacterial effect is greater than the sum of the effects exerted by the individual substances. Furthermore, the present composition is able to simultaneously promote the growth of the lactobacilli, naturally present in the vagina.

The composition according to the present invention may be used as a therapeutic agent for treating the bacterial vaginosis.

In the forms of therapeutic use, the composition according to the present invention may be applied to the vaginal mucosa in the form of a liquid solution, in solid form as ovules or semi-solid as cream.

The dosage for therapeutic use of the composition in the form of solid ovules may be one to two 1.5 - 2.5 g ovules/day in the vagina.

The dosage for therapeutic use of the liquid composition may be by douching with the contents of a 100 ml bottle one to two times a day. In the case of cream applications according to the present invention, the creams may be applied one or two times a day using the 5 g doser provided.

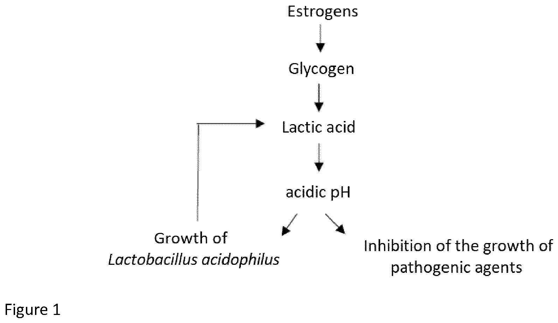

Figure 1 represents the virtuous circle that is established in the vagina in physiological conditions.

DESCRIPTION OF THE EMBODIMENTS OF THE INVENTION

The main constituents of the composition are reported below together with the description of their functions and the rational of their choice.

The term "dequalinium salt" as used herein means a 1,1'-decamethylenebis(4-aminoquinaldinium) salt. In the dequalinium salt, cation 1,1'-decamethylenebis(4-aminoquinaldinium) is pharmaceutically active, while the salt may contain any pharmaceutically acceptable anion, such as chloride, bromide, iodide, acetate or undecanoate. The term "dequalinium ion" as used herein means the cation 1,1'-decamethylenebis(4-aminoquinaldinium).

According to a preferred embodiment, the dequalinium salt is dequalinium chloride (DQC).

The DQC is an antiseptic indicated for the topical treatment of the BV. The DQC is a quaternary ammonium salt that has a wide antimicrobial spectrum ranging from Gram+ to Gram-, fungi and some protozoa. It is effective against the main pathogens responsible for the vaginal infections.

The mechanism of action of the DQC is firstly based on its effects on the permeability of the bacterial cells: the DQC is adsorbed on the bacterial cell wall and diffuses inside it where it causes the denaturation of the proteins involved in the respiratory chain and glycolysis inhibiting the main metabolic reactions. According to the concentration, the dequalinium chloride also causes an osmotic imbalance that leads to lysis of the cell of the pathogenic agent. Thanks to this multiple mechanism of action, the DQC has a much wider antibacterial action spectrum than many antibiotics and has up to now prevented the creation of resistant bacterial strains.

The DQC is also effective against the relapses and, thanks to the meagre systemic absorption, it can be used in pregnancy and breastfeeding (Vera Della Casa, Harald Noll, Susanne Gonsera, Philipp Groba, Federico Graf and Gabriele Pohlig, "Antimicrobial Activity of Dequalinium Chloride against Leading Germs of Vaginal Infections", Arzneim.-Forsch./Drug Res. 2002, 52, No. 9,699-705).

In light of these considerations, the DQC is incorporated into the composition because of its local disinfectant action that facilitates the eradication of the pathogenic bacteria that cause the BV.

The maltodextrins represent the nutritious substrate of the lactobacilli adapted to promote their growth. The maltodextrins are effective in promoting the proliferation of the lactobacilli in the presence of lactic acid and in reestablishing the optimal vaginal ecosystem.

In light of the present considerations, the maltodextrins are incorporated into the composition because of their ability to act as a metabolic substrate for the lactobacilli without stimulating the growth of other undesired micro-organisms.

The lactic acid is a carboxylic acid that performs a significant role in different biochemical processes. It is produced in the vagina by the lactobacillus flora starting from glycogen in order to keep the vaginal pH at that degree of acidity (3.5-4.5) which allows an actual barrier to be actuated against any pathogenic micro-organisms. Such function is widely known and documented (Haya J., Garcia A., López-Manzanara C., Balawi M., Haya L., "Importance of Lactic Acid in Maintaining Vaginal Health: A Review of Vaginitis and Vaginosis Etiopathogenic Bases and a Proposal for a New Treatment", Open Journal of Obstetrics and Gynecology, 2014, 4, 787-799).

The administration of lactic acid leads to a lowering of the pH in the vagina, triggering a virtuous protective circuit for the vaginal environment: the lactobacilli in turn produce lactic acid that contributes to lowering the pH further, creating an unfavourable environment to the growth of pathogenic agents and at the same time favourable to the development of *Lactobacillus acidophilus* **(Figure 1).** Furthermore, the lactobacilli produce bacteriocins and hydrogen peroxide with antimicrobial activity. This effective defence mechanism leads to the resolution of the vaginal disorders.

In light of the present considerations, the lactic acid is incorporated into the composition due to its ability to lower the pH and promote the recolonisation of the commensal lactobacilli. According to a preferred embodiment, the composition is a solid composition in the form of ovules and the quantity of dequalinium ion is between 1.7 mg and 26 mg per 2 g of product, preferably the quantity of dequalinium ion is between 4.4 mg and 26 mg per 2 g of product, more preferably the quantity of dequalinium ion is equal to 6.9 mg. Still according to this embodiment, the quantity of maltodextrin is between 2 mg and 9 mg per 2 g of product, preferably the quantity of maltodextrin is equal to 5 mg. Still according to this embodiment, the quantity of lactic acid is between 2 mg and 40 mg per 2 g of product, preferably the quantity of lactic acid is equal to 10 mg.

According to a further embodiment, the composition is a semi-solid composition in the form of cream and the quantity of dequalinium ion is between 1.7 mg and 26 mg per 5 g of product, preferably the quantity of dequalinium ion is between 4.4 mg and 26 mg per 5 g of product, more preferably the quantity of dequalinium ion is equal to 6.9 mg.

Still according to this embodiment, the quantity of maltodextrin is between 2 mg and 9 mg per 5 g of product, preferably the quantity of maltodextrin is equal to 5 mg. Still according to this embodiment, the quantity of lactic acid is between 2 mg and 40 mg per 5 g of product, preferably the quantity of lactic acid is equal to 10 mg.

According to a preferred embodiment the composition is a liquid composition and the quantity of dequalinium ion is between 1.7 mg and 26 mg per 100 ml of solution, preferably the quantity of dequalinium ion is between 4.4 mg and 26 mg per 100 ml of solution, more preferably the quantity of dequalinium ion is equal to 6.9 mg. Still according to this embodiment, the quantity of maltodextrin is between 2 mg and 9 mg per 100 ml of solution, preferably the quantity of maltodextrin is equal to 5 mg. Still according to this embodiment, the quantity of lactic acid is between 2 mg and 40 mg per 100 ml of solution, preferably the quantity of lactic acid is equal to 10 mg.

A particularly preferred mode of the composition in the solid formula (ovules) contains the following quantities of substances per 2 g:

8 mg of dequalinium chloride, 5 mg of maltodextrin and 10 mg of lactic acid.

A particularly preferred mode of the composition in the semi-solid formula (cream) contains the following quantities of substances per 5 g:

8 mg of dequalinium chloride, 5 mg of maltodextrin and 10 mg of lactic acid.

A particularly preferred mode of the composition in the liquid form contains the following quantities of substances per 100 ml:

8 mg of dequalinium chloride, 5 mg of maltodextrin and 10 mg of lactic acid.

**Experimental part**

The experimental part was performed for the purpose of reaching the objective set in the present patent application and was organised through two tests; the first aims to identify the optimal concentration of lactic acid for restoring the physiological vaginal pH and the second aims to demonstrate the synergistic activity of dequalinium chloride, maltodextrin and lactic acid used in combination to inhibit the growth of the main bacteria involved in the onset of the BV and simultaneously promote the growth of the endogenous lactobacilli.

**Test 1: Identification of the optimal concentration of lactic acid**

As the maintenance of an acid vaginal pH spontaneously stimulates the proliferation of the lactobacilli preventing the growth of pathogens, the present test was performed to verify the optimal concentration of lactic acid for lowering the pH to physiological levels (3.5-4.5).

*Test 1A: ovules containing 1% lactic acid (20 mg/dose)*

The inventors decided to test a 20 mg quantity of lactic acid and, possibly, to change the dose based on the pH values recorded.

Method

A pilot test was performed obtaining ovules containing the following quantities of raw materials:

| Raw material | Content per ovule (mg) |
|---|---|
| Semi-synthetic glycerides DUB*** | 1965 |
| Lactic acid 90%* | 22 |
| Maltodextrin | 5 |
| Dequalinium chloride** | 8 |
| TOTAL | 2000 |
| *corresponding to 20 mg of 100% lactic acid. ** with 100% titre restoration. *** component compensating for the titre restoration | |

2 tests were performed by suspending an ovule in a beaker containing 150 ml of water under magnetic agitation at 37°C +/- 0.5°C and buffered to pH 5.5 through $NaH_2PO_4$ and NaOH. The pH was measured continuously every 30 seconds until the normalisation of the effect. Any value comprised in the pH range present in physiological conditions in the vagina (3.5-4.5) is considered an optimal target pH.

Results and Discussion

Table 1 summarises the pH values recorded.

| Time (s) | pH 1st ovule | pH 2nd ovule |
|---|---|---|
| 00:00 | 5.36 | 5.28 |
| 00:30 | 4.31 | 5.30 |
| 01:00 | 3.86 | 4.03 |
| 01:30 | 3.67 | 3.79 |
| 02:00 | 3.56 | 3.60 |
| 02:30 | 3.49 | 3.65 |

(continued)

| Time (s) | pH 1st ovule | pH 2nd ovule |
|----------|--------------|--------------|
| 03:00 | 3.44 | 3.64 |
| 03:30 | 3.37 | 3.64 |
| 04:00 | 3.38 | 3.63 |
| 04:30 | 3.36 | 3.61 |
| 05:00 | 3.35 | 3.61 |
| 05:30 | 3.33 | 3.60 |
| 06:00 | 3.32 | 3.60 |
| 06:30 | 3.31 | 3.59 |
| 07:00 | 3.31 | 3.58 |
| 07:30 | 3.31 | 3.58 |
| 08:00 | 3.30 | 3.57 |
| 08:30 | 3.30 | 3.57 |
| 09:00 | 3.30 | 3.57 |
| 09:30 | 3.30 | 3.56 |
| 10:00 | 3.31 | 3.56 |
| 10:30 | 3.31 | 3.55 |
| **Table 1:** pH values recorded after dissolution of the two ovules (ref. pilot test 201720). | | |

From the analysis of the results it can be deduced that the acidifying effect of the dosing unit prepared with pilot test no. 201720 was rather drastic, particularly in the first minute of dissolution. The final pH of 3.3 reached was considered too low as it did not fall within the pH range present in physiological conditions in the vagina.

Therefore, it was decided to perform a second test on ovules in which the concentration of lactic acid is reduced by 50% (see Test 1B).

*Test 1B: ovules containing 0.5 % lactic acid (10 mg/dose)*

Method

Two pilot tests were performed obtaining ovules containing the following quantities of raw materials:

| Raw material | Content per ovule (mg) |
|--------------|------------------------|
| Semi-synthetic glycerides DUB*** | 1976 |
| Lactic acid 90%* | 11 |
| Maltodextrin | 5 |
| Dequalinium chloride** | 8 |
| TOTAL | 2000 |
| *corresponding to 10 mg of 100% lactic acid. ** with 100% titre restoration. *** component compensating for the titre restoration | |

The test is performed on two ovules taken from each of the pilot tests 201723 and 201725 following the procedure described in Test 1A.

Results and Discussion

Table 2 summarises the pH values recorded.

| Time (s) | pH 1st ovule* | pH 2nd ovule* | pH 1st ovule** | pH 2nd ovule** |
|---|---|---|---|---|
| 00:00 | 4.93 | 5.99 | 5.39 | 5.44 |
| 00:30 | 4.93 | 5.99 | 5.27 | 5.25 |
| 01:00 | 4.94 | 4.88 | 5.06 | 5.11 |
| 01:30 | 4.93 | 4.35 | 4.57 | 4.65 |
| 02:00 | 4.48 | 4.17 | 4.28 | 4.41 |
| 02:30 | 4.05 | 4.01 | 4.09 | 4.26 |
| 03:00 | 3.86 | 3.94 | 3.99 | 4.20 |
| 03:30 | 3.75 | 3.89 | 3.92 | 4.17 |
| 04:00 | 3.69 | 3.84 | 3.88 | 4.14 |
| 04:30 | 3.66 | 3.81 | 3.84 | 4.14 |
| 05:00 | 3.63 | 3.78 | 3.81 | 4.09 |
| 05:30 | 3.61 | 3.74 | 3.79 | 4.09 |
| 06:00 | 3.60 | 3.71 | 3.78 | 4.09 |
| 06:30 | 3.59 | 3.69 | 3.77 | 4.07 |
| 07:00 | 3.58 | 3.68 | 3.76 | 4.06 |
| 07:30 | 3.57 | 3.65 | 3.76 | 4.06 |
| 08:00 | 3.57 | 3.63 | 3.76 | 4.06 |
| 08:30 | 3.56 | 3.62 | 3.75 | 4.05 |
| 09:00 | 3.56 | 3.61 | 3.75 | 4.05 |
| 09:30 | 3.56 | 3.59 | 3.75 | 4.04 |
| 10:00 | 3.56 | 3.59 | 3.75 | 4.04 |
| 10:30 | 3.57 | 3.58 | 3.75 | 4.04 |
| 11:00 | 3.57 | 3.57 | 3.75 | 4.04 |
| 11:30 | 3.57 | 3.57 | 3.75 | 4.04 |
| 12:00 | 3.57 | 3.56 | 3.75 | 4.04 |
| 12:30 | 3.56 | 3.56 | 3.75 | 4.04 |
| 13:00 | 3.56 | 3.55 | 3.76 | 4.04 |
| 13:30 | 3.56 | 3.55 | 3.76 | 4.04 |
| 14:00 | 3.57 | 3.55 | 3.76 | 4.04 |
| 14:30 | 3.57 | 3.54 | 3.76 | 4.04 |
| 15:00 | 3.57 | 3.54 | 3.76 | 4.04 |
| 15:30 | 3.57 | 3.54 | 3.75 | 4.04 |
| 16:00 | 3.57 | 3.54 | 3.75 | 4.04 |
| 16:30 | 3.57 | 3.54 | 3.75 | 4.05 |
| 17:00 | 3.57 | 3.53 | 3.75 | 4.05 |

(continued)

| Time (s) | pH 1st ovule* | pH 2nd ovule* | pH 1st ovule** | pH 2nd ovule** |
|---|---|---|---|---|
| 17:30 | 3.57 | 3.53 | 3.75 | 4.05 |
| 18:00 | 3.58 | 3.53 | 3.75 | 4.05 |
| 18:30 | 3.58 | 3.53 | 3.75 | 4.05 |
| 19:00 | 3.58 | 3.53 | 3.75 | 4.05 |
| 19:30 | 3.58 | 3.53 | 3.76 | 4.05 |
| 20:00 | 3.58 | 3.53 | 3.76 | 4.05 |
| **Table 2:** pH values recorded after dissolution of the two ovules taken from each of the pilot tests 201723* and 201725**. | | | | |

From the analysis of the results it can be deduced that the acidifying effect of the dosing units prepared with the pilot tests of table 2 was less drastic than the test of table 1. The effect is in line with expectations; in fact, the final pH measured varies between about 3.5 and 4.0, values comprised in the pH range present in physiological conditions in the vagina.

Conclusions

From the analysis of the results obtained in Tests 1A and 1B described above, it can be deduced that the optimal concentration of lactic acid per ovule (2 g) is 10 mg.

**Test 2: Verification of the synergistic activity of a composition based on dequalinium chloride, maltodextrin and lactic acid**

The test consists of demonstrating:

- that the composition of dequalinium chloride, maltodextrin and lactic acid is able to inhibit the growth of some of the main bacteria responsible for the BV and that these substances perform a synergistic action i.e. that their combination is able to exert a greater antibacterial effect than the sum of the effects exerted by the individual ingredients;
- that the composition of dequalinium chloride, maltodextrin and lactic acid is able to simultaneously promote the proliferation of the endogenous lactobacilli that represent in themselves a first defence of the organism against the growth of the pathogenic micro-organisms. The selectivity of the compostion is verified by evaluating the survival ability of the *Lactobacillus Acidophilus* in the presence and absence of another bacterial strain in metabolic competition.

The activity of the composition of dequalinium chloride, maltodextrin and lactic acid **(DQC+MD+AL)** is compared with:

- dequalinium chloride used alone (**DQC**)
- maltodextrin and lactic acid (**MD+AL**) in combination as it is stated (in patent IT2003T000404) that lactic acid has a positive effect on the activity of the maltodextrins in promoting the growth of the lactobacilli to the detriment of the pathogenic micro-organisms.

Strains tested

- Escherichia coli ATCC 8739
- Staphylococcus aureus ATCC 6538
- Prevotella loescheii ATCC 15930
- Gardnerella vaginalis ATCC 11418
- Lactobacillus Acidophilus ATCCC 4356
- Gardnerella vaginalis ATCC 11418 + Lactobacillus Acidophilus ATCCC 4356

Culture media

- CCA agar for the culture of Escherichia coli
- M.R.S. agar for the culture of Lactobacillus Acidophilus (anaerobiosis)
- Mannitol Salt agar for the culture of Staphylococcus aureus
- Blood agar sheep for the culture of Prevotella loescheii
- Chocolate enriched agar for the culture of Gardnerella vaginalis

Method

3 different mixtures of substances were prepared whose composition is described below:

- **MD** (5 mg) **+ AL** (90%, 11 mg)

- **DQC** (8 mg)

- **DQC** (8 mg) **+MD** (5 mg) **+AL** (90%, 11 mg)

To evaluate the antibacterial efficacy of the ingredients in the three compositions described above, the pure raw materials directly dissolved in the culture medium were tested. Aliquots of the 3 mixtures were prepared for each of the bacterial strains being evaluated. Each mixture was placed in a receptacle containing a suitable volume of a peptone water solution with pH 5.5 having a concentration of the bacterial strain equal to at least $10^5$ CFU/ml.

Each container was placed in incubation at 37°C for 4 hours to promote the dissolution and the action of the ingredients. A negative control was prepared for each strain i.e. a solution without the presence of the substances being tested, which is treated in the same experimental conditions as the receptacles containing the mixtures being tested.

At the end of the incubation period the contents of each container were neutralised and seeded in a suitable culture medium, the micro-organisms that had grown were counted. Each seeding was performed in triplicate.

Results and Discussion

Table 3 shows the results obtained. The survival level (expressed in CFU/ml) of each bacterial strain tested and the percentage reduction of the microbial load with respect to the one recorded in the negative control is stated for each mixture.

**Table 3:** Reduction in the microbial load after treatment with the threecompositions.

| Strain | Sample | Microbial load (CFU/ml)* | Reduction in microbial load with respect to the negative control |
|---|---|---|---|
| Escherichia coli | Negative control | 2,100,000 | / |
| | MD+AL | 1,673,070 | 20.33% |
| | DQC | 1,037,400 | 50.60% |
| | DQC + MD + AL | 13,567 | 99.35% |

(continued)

| Strain | Sample | Microbial load (CFU/ml)* | Reduction in microbial load with respect to the negative control |
|---|---|---|---|
| Staphylococcus aureus | Negative control | 35,667 | / |
| | MD+AL | 28,480 | 20.15% |
| | DQC | 14,267 | 60.00% |
| | DQC + MD + AL | 6,716 | 81.17% |
| Prevotella loescheii | Negative control | 334,333 | / |
| | MD+AL | 309,759 | 7.35% |
| | DQC | 3,367 | 90.56% |
| | DQC + MD + AL | 180 | 99.95% |
| Lactobacillus Acidophilus | Negative control | 138.00 | / |
| | MD+AL | 96,667 | 29.95% |
| | DQC | 5,267 | 96.18% |
| | **DQC + MD + AL** | **159.333** | **-15.46%** |
| Gardnerella vaginalis | Negative control | 16,367 | / |
| | MD+AL | 15,547 | 5.00% |
| | DQC | 4,205 | 74.31% |
| | DQC + MD + AL | 1,610 | 90.16% |
| Lactobacillus Acidophilus + Gardnerella vaginalis** | Negative control | 3,500 | / |
| | MD+AL | 5,400 | -54.29% |
| | DQC | 1,400 | 60.00% |
| | **DQC + MD + AL** | **8,600** | **-145.71%** |
| * The stated value is the mean of the values obtained in the 3 replicates. **The stated values only refer to the lactobacilli. To distinguish between the two bacterial strains, each mixture was seeded in media selective to the lactobacilli. | | | |

From the analysis of the results it can be deduced that:

- the mixture comprising dequalinium chloride (8 mg), maltodextrin (5 mg) and lactic acid (90%, 11 mg) exerts a synergistic effect on the main bacteria responsible for the BV (Escherichia coli, Staphylococcus aureus, Prevotella loescheii, Gardnerella vaginalis). In fact, the inhibitory effect of the DQC + MD + AL mixture on the bacterial proliferation is greater than the sum of the effect exerted by the ingredients used individually.
- the DQC + MD + AL mixture is able to promote the proliferation of the endogenous lactobacilli present in the vagina by 15.46%. Such positive effect on the growth of the so-called "good" bacteria was not found for DQC or for the MD + AL mixture; in fact DQC and MD + AL cause an inhibition of the proliferation of the lactobacilli, of 96.18% and 29.95%, respectively.

- the DQC + MD + AL mixture is able to promote the growth of the lactobacilli also in the presence of a pathogenic bacterial strain, such as the Gardnerella vaginalis, in metabolic competition with the Lactobacillus Acidophilus. Such effect is almost three times greater than that exerted by the MD + AL mixture. In fact, the DQC + MD + AL sample increases the proliferation of the lactobacilli by 145.71%, whereas for the MD + AL mixture, only a 54.29% increase was observed. Instead, the sample only containing dequalinium determines 60.00% inhibition of the growth of the lactobacilli.

Similar results were obtained with aqueous solutions or with the use of creams according to the invention.

From the analysis of the results described above it can be deduced that the combination of dequalinium chloride (8 mg), maltodextrin (5 mg) and lactic acid (90%, 11 mg) represents an effective therapy for the eradication of the BV, and better than some treatments used in the gynaecological field. In fact, the DQC + MD + AL mixture performs a double effect, it inhibits the growth of pathogenic bacteria and, simultaneously, determines an increase in the proliferation of the endogenous lactobacilli that represent in themselves a first defence of the organism against the growth of the pathogenic micro-organisms. Both the effects exerted by the DQC + MD + AL mixture are greater than those observed for the individual ingredients.

## Claims

1.  Composition comprising a dequalinium salt, maltodextrin and lactic acid for use in the treatment of bacterial vaginosis (BV).

2.  The composition for use
    according to claim 1, wherein the anion of the dequalinium salt is selected from chloride, bromide, iodide, acetate or undecanoate; preferably chloride.

3.  The composition for use
    according to claim 1 or 2, wherein the composition is a solid
    composition in the form of ovules, preferably the concentration by weight of dequalinium ion is between 0.85‰ - 13‰, preferably 2.20‰ - 13‰, more preferably 3.45‰ - 9‰.

4.  The composition for use
    according to claim 3, wherein the concentration by weight of maltodextrin is between 1.0‰ - 4.5‰, preferably 2.0‰ - 3.0‰.

5.  The composition for use
    according to claim 3 or 4, wherein the lactic acid concentration by weight is between 1.0‰ - 20‰, preferably 4.0‰ - 6,0‰.

6.  The composition for use
    according to any one of claims 3 to 5, wherein the ovules have a weight of 1.5-2.5 g and optionally comprise one or more stable pharmaceutically acceptable excipients selected from semi-synthetic glycerides, silica dimethyl silylate, vitamin E acetate and butylhydroxytoluene; preferably the ovules have a weight of 2 g.

7.  The composition for use
    according to claim 1 or 2, wherein the composition is an aqueous
    composition optionally comprising one or more stable pharmaceutically acceptable excipients; preferably the concentration by weight of dequalinium ion is between 0.017‰ - 0.26‰, the concentration by weight of maltodextrin is between 0,02‰ - 0.09‰, and the concentration by weight of lactic acid is between 0.02‰ - 0.4‰; more preferably the concentration by weight of dequalinium ion is between 0.044‰ - 0.26‰, the concentration by weight of maltodextrin is between 0.04‰ - 0.06‰, and the concentration by weight of lactic acid is between 0.08‰ - 0.12‰; most preferably the dequalinium ion concentration by weight is between 0.069‰ - 0.18‰, maltodextrin between 0.04‰ and 0.06‰, and lactic acid between 0.08‰ and 0.12‰.

8.  The composition for use
    according to claim 7, wherein the stable pharmaceutically
    acceptable excipients are selected from glycerin, PEG 40 hydrogenated castor oil, chlorhexidine digluconate, sodium benzoate, lavender essential oil; preferably the aqueous composition is packaged in disposable 100 ml bottles.

9. The composition for use
according to claim 1 or 2, wherein the composition is a water-based cream optionally comprising one or more stable pharmaceutically acceptable excipients; preferably the concentration by weight of dequalinium ion is between 0.34‰ - 5.2‰, the concentration by weight of maltodextrin is between 0.4‰ - 1.8‰o, the concentration by weight of lactic acid is between 0.4‰ - 8‰; more preferably the concentration by weight of dequalinium ion is between 0.88‰ and 5.2‰, the concentration by weight of maltodextrin is between 0.8‰ - 1.2‰, the lactic acid concentration by weight is between 1.6‰ - 2.4‰; most preferably the concentration by weight of dequalinium ion is between 1.38‰ - 3.6‰, maltodextrin between 0.8‰ - 1.2‰, and lactic acid is between 1.6‰ - 2.4‰.

10. The composition for use
according to claim 9, wherein the stable pharmaceutically
acceptable excipients of the cream are selected from one or more excipients selected from glyceryl stearate, PEG 100 stearate, cetylstearyl alcohol, fatty acid esters, cyclopentasiloxane, vegetable stearic acid, chlorhexidine digluconate, silicone oil, triethanolamine, EDTA bisodic salt, carbomer and other lipophilic excipients; preferably the cream is packaged in 30 g tubes with 6 disposable applicators.


## Patentansprüche

1. Zusammensetzung, umfassend ein Dequaliniumsalz, Maltodextrin und Milchsäure zur Verwendung bei der Behandlung von bakterieller Vaginose (BV).

2. Zusammensetzung zur Verwendung nach Anspruch 1, wobei das Anion des Dequaliniumsalzes ausgewählt ist aus Chlorid, Bromid, Iodid, Acetat oder Undecanoat; bevorzugt Chlorid.

3. Zusammensetzung zur Verwendung nach Anspruch 1 oder 2, wobei die Zusammensetzung eine feste Zusammensetzung in Form von Ovula ist, wobei die Gewichtskonzentration des Dequaliniumions bevorzugt zwischen 0,85‰ - 13‰, bevorzugt 2,20‰ - 13‰, bevorzugter 3,45‰ - 9‰, liegt.

4. Zusammensetzung zur Verwendung nach Anspruch 3, wobei die Gewichtskonzentration von Maltodextrin zwischen 1,0‰ - 4,5‰, bevorzugt 2,0‰ - 3,0‰, liegt.

5. Zusammensetzung zur Verwendung nach Anspruch 3 oder 4, wobei die Milchsäurekonzentration, bezogen auf das Gewicht, zwischen 1,0‰ - 20‰, bevorzugt 4,0‰ - 6,0‰, liegt.

6. Zusammensetzung zur Verwendung nach irgendeinem der Ansprüche 3 bis 5, wobei die Ovula ein Gewicht von 1,5 - 2,5 g aufweisen und gegebenenfalls einen oder mehrere stabile pharmazeutisch verträgliche Exzipienten umfassen, ausgewählt aus halbsynthetischen Glyceriden, Siliciumdioxid Dimethylsilylat, Vitamin E Acetat und Butylhydroxytoluol; wobei die Ovula bevorzugt ein Gewicht von 2 g aufweisen.

7. Zusammensetzung zur Verwendung nach Anspruch 1 oder 2, wobei die Zusammensetzung eine wässrige Zusammensetzung ist, die gegebenenfalls einen oder mehrere stabile pharmazeutisch verträgliche Exzipienten umfasst; wobei es bevorzugt ist, dass die Gewichtskonzentration des Dequaliniumions zwischen 0,017‰ - 0,26‰ liegt, die Gewichtskonzentration von Maltodextrin zwischen 0,02‰ - 0,09‰ liegt und die Gewichtskonzentration von Milchsäure zwischen 0,02‰ - 0,4‰ liegt; wobei es bevorzugter ist, dass die Gewichtskonzentration des Dequaliniumions zwischen 0,044‰ - 0,26‰ liegt, die Gewichtskonzentration von Maltodextrin zwischen 0,04‰ - 0,06‰ liegt und die Gewichtskonzentration von Milchsäure zwischen 0,08‰ - 0,12‰ liegt; wobei es am meisten bevorzugt ist, dass die Gewichtskonzentration an Dequaliniumion zwischen 0,069‰ - 0,18‰, an Maltodextrin zwischen 0,04‰ und 0,06‰ und an Milchsäure zwischen 0,08‰ und 0,12‰ liegt.

8. Zusammensetzung zur Verwendung nach Anspruch 7, wobei die stabilen pharmazeutisch verträglichen Exzipienten ausgewählt sind aus Glycerin, PEG 40 hydriertem Kastoröl, Chlorhexidindigluconat, Natriumbenzoat, ätherischem Lavendelöl; wobei die wässrige Zusammensetzung bevorzugt in 100 ml Einwegflaschen verpackt ist.

9. Zusammensetzung zur Verwendung nach Anspruch 1 oder 2, wobei die Zusammensetzung eine Creme auf Wasserbasis ist, die gegebenenfalls einen oder mehrere stabile pharmazeutisch verträgliche Exzipienten umfasst; wobei es bevorzugt ist, dass die Gewichtskonzentration an Dequaliniumion zwischen 0,34‰ - 5,2‰ liegt, die Gewichtskonzentration an Maltodextrin zwischen 0,4‰ - 1,8‰ liegt, die Gewichtskonzentration an Milchsäure zwischen 0,4‰ - 8‰ liegt; wobei es bevorzugter ist, dass die Gewichtskonzentration an Dequaliniumion zwischen 0,88‰ und 5,2‰ liegt, die Gewichtskonzentration an Maltodextrin zwischen 0,8‰ - 1,2‰ liegt, die Gewichtskonzentration an Milchsäure zwischen 1,6‰ - 2,4‰ liegt; wobei es am meisten bevorzugt ist, dass die Gewichtskonzentration an Dequaliniumion zwischen 1,38‰ - 3,6‰ liegt, an Maltodextrin zwischen 0,8‰ - 1,2‰ liegt und an Milchsäure zwischen 1,6‰ - 2,4‰ liegt.

10. Zusammensetzung zur Verwendung nach Anspruch 9, wobei die stabilen pharmazeutisch verträglichen Exzipienten der Creme ausgewählt sind aus einem oder mehreren Exzipienten, ausgewählt aus Glycerylstearat, PEG 100-Stearat, Cetylstearylalkohol, Fettsäureestern, Cyclopentasiloxan, pflanzlicher Stearinsäure, Chlorhexidindigluconat, Siliconöl, Triethanolamin, EDTA-Binatriumsalz, Carbomer und anderen lipophilen Exzipienten; wobei die Creme bevorzugt in 30 g Röhrchen mit 6 Einwegapplikatoren verpackt ist.

## Revendications

1. Composition comprenant un sel de déqualinium, de la maltodextrine et de l'acide lactique pour utilisation dans le traitement de la vaginose bactérienne (BV).

2. Composition pour utilisation selon la revendication 1, dans laquelle l'anion du sel de déqualinium est sélectionné parmi du chlorure, du bromure, de l'iodure, de l'acétate ou de l'undécanoate ; de préférence du chlorure.

3. Composition pour utilisation selon la revendication 1 ou 2, dans laquelle la composition est une composition solide sous la forme d'ovules, de préférence la concentration en poids d'ion de déqualinium est entre 0,85 ‰ et 13 ‰, de préférence entre 2,20 ‰ et 13 ‰, plus préférablement entre 3,45 ‰ et 9 ‰.

4. Composition pour utilisation selon la revendication 3, dans laquelle la concentration en poids de maltodextrine est entre 1,0 ‰ et 4,5 ‰, de préférence entre 2,0 ‰ et 3,0 ‰.

5. Composition pour utilisation selon la revendication 3 ou 4, dans laquelle la concentration en poids d'acide lactique est entre 1,0 ‰ et 20 ‰, de préférence entre 4,0 ‰ et 6,0 ‰.

6. Composition pour utilisation selon l'une quelconque des revendications 3 à 5, dans laquelle les ovules présentent un poids de 1,5 à 2,5 g et comprennent optionnellement un ou plusieurs excipients stables pharmaceutiquement acceptables sélectionnés parmi des glycérides semi-synthétiques, du diméthylsilylate de silice, de l'acétate de vitamine E et du butylhydroxytoluène ; de préférence les ovules présentent un poids de 2 g.

7. Composition pour utilisation selon la revendication 1 ou 2, dans laquelle la composition est une composition aqueuse comprenant optionnellement un ou plusieurs excipients stables pharmaceutiquement acceptables ; de préférence la concentration en poids d'ion de déqualinium est entre 0,017‰ et 0,26‰, la concentration en poids de maltodextrine est entre 0,02 ‰ et 0,09 ‰, et la concentration en poids d'acide lactique est entre 0,02 ‰ et 0,4 ‰ ; plus préférablement la concentration en poids d'ion de déqualinium est entre 0,044 ‰ et 0,26 ‰ la concentration en poids de maltodextrine est entre 0,04 ‰ et 0,06 ‰, et la concentration en poids d'acide lactique est entre 0,08 ‰ et 0,12 ‰ ; idéalement la concentration en poids d'ion de déqualinium est entre 0,069 ‰ et 0,18 ‰, de maltodextrine entre 0,04 ‰ et 0,06 ‰ et d'acide lactique entre 0,08 ‰ et 0,12 ‰.

8. Composition pour utilisation selon la revendication 7, dans laquelle les excipients stables pharmaceutiquement acceptables sont sélectionnés parmi la glycérine, l'huile de ricin hydrogénée PEG 40, le digluconate de chlorhexidine, le benzoate de sodium, l'huile essentielle de lavande ; de préférence la composition aqueuse est conditionnée dans des bouteilles jetables de 100 ml.

9. Composition pour utilisation selon la revendication 1 ou 2, dans laquelle la composition est une crème à base d'eau comprenant optionnellement un ou plusieurs excipients stables pharmaceutiquement acceptables ; de préférence la concentration en poids d'ion de déqualinium est entre 0,34 ‰ et 5,2 ‰, la concentration en poids de maltodextrine est entre 0,4 ‰ et 1,8 ‰, la concentration en poids d'acide lactique est entre 0,4 ‰ et 8 ‰ ; plus préférablement la concentration en poids d'ion de déqualinium est entre 0,88 ‰ et 5,2 ‰, la concentration en poids de maltodextrine est entre 0,8 ‰ et 1,2 ‰, la concentration en poids d'acide lactique est entre 1,6 ‰ et 2,4 ‰ ; idéalement la concentration en poids d'ion de déqualinium est entre 1,38 ‰ et 3,6 ‰, de maltodextrine entre 0,8 ‰ et 1,2 ‰, et d'acide lactique entre 1,6 ‰ et 2,4 ‰

**10.** Composition pour utilisation selon la revendication 9, dans laquelle les excipients stables pharmaceutiquement acceptables de la crème sont sélectionnés parmi un ou plusieurs excipients sélectionnés parmi le stéarate de glycéryle, le stéarate PEG 100, l'alcool de cétylstéaryle, des esters d'acides gras, le cyclopentasiloxane, l'acide stéarique végétal, le digluconate de chlorhexidine, l'huile de silicone, la triéthanolamine, un sel disodique EDTA, un carbomère et d'autres excipients lipophiles ; de préférence la crème est conditionnée dans des tubes de 30 g avec 6 applicateurs jetables.

Estrogens

↓

Glycogen

↓

Lactic acid

↓

acidic pH

Growth of
*Lactobacillus acidophilus*

Inhibition of the growth of
pathogenic agents

Figure 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IT 2003TO00404
- EP 1481666 A
- US 3147182 A
- GB 1126953 A
- GB 2450225 A
- EP 2152264 A

**Non-patent literature cited in the description**

- **HAYA J. ; GARCIA A. ; LÓPEZ-MANZANARA C. ; BALAWI M. ; HAYA L.** Importance of Lactic Acid in Maintaining Vaginal Health: A Review of Vaginitis and Vaginosis Etiopathogenic Bases and a Proposal for a New Treatment. *Open Journal of Obstetrics and Gynecology,* 2014, vol. 4, 787-799
- **CAROL A. SPIEGEL.** Bacterial Vaginosis. *Clinical Microbiology Reviews,* October 1991, 485-502
- Use of locally delivered dequalinium chloride in the treatment of vaginal infections : a review. **W. MENDLING et al.** Archives of Gynecology and Obstetrics. Springer Verlag, 27 October 2015, vol. 293, 469-484
- **VERA DELLA CASA ; HARALD NOLL ; SUSANNE GONSERA ; PHILIPP GROBA ; FEDERICO GRAF ; GABRIELE POHLIG.** Antimicrobial Activity of Dequalinium Chloride against Leading Germs of Vaginal Infections. *Arzneim.-Forsch./Drug Res.,* 2002, vol. 52 (9), 699-705